# EUROPEAN PATENT APPLICATION

(11) **EP 4 729 065 A1**
(43) Date of publication of application: **22.04.2026**
(21) Application number: 24822805.8
(22) Date of filing: 14.06.2024
(51) Int. Cl.: A61K 38/08, A61K 9/20, A61P 25/04, A61P 17/04

(54) **ORAL PHARMACEUTICAL COMPOSITION OF PEPTIDE AMIDE COMPOUND AND PREPARATION METHOD THEREFOR**

(30) Priority: 15.06.2023 CN 202310707192
(71) Applicant: Haisco Pharmaceutical Group Co., Ltd., Shannan, Tibet 856000 (CN)
(72) Inventor: LI, Xiaoping, Chengdu, Sichuan 611130 (CN); FU, Ling, Chengdu, Sichuan 611130 (CN); LIU, Bo, Chengdu, Sichuan 611130 (CN); GUAN, Lin, Chengdu, Sichuan 611130 (CN); LIU, Xiangdong, Chengdu, Sichuan 611130 (CN)
(74) Representative: AWA Denmark A/S
(86) International application number: PCT/CN2024/099292
(87) International publication number: WO 2024/255858

(57) **Abstract**

An oral pharmaceutical composition of a peptide amide compound A for treating pain and pruritus and a preparation method therefor. The composition comprises compound A and a microcrystalline cellulose colloidal silica co-treatment substance, and further comprises a disintegrant and/or a lubricant. The composition can be prepared into an oral preparation form such as a tablet or a capsule.

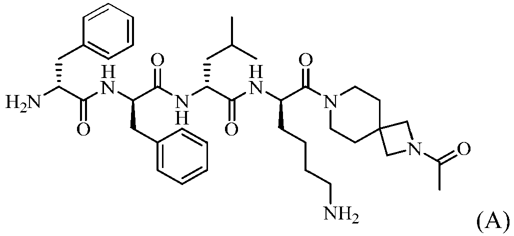

## Description

### Technical Field

The present invention relates to the field of chemical pharmaceutical technology, and in particular to an oral pharmaceutical composition of a peptide amide compound (A) for treating pain and pruritus, and a method for preparing such a pharmaceutical composition for treating pain and pruritus and a packaged combination thereof.

### Background Art

Pain has been identified by the World Health Organization as the fifth vital sign, alongside respiration, pulse, blood pressure, and body temperature. The International Association for the Study of Pain (IASP) proposed the current internationally accepted definition of pain in 1994: an unpleasant sensory and emotional experience associated with, or resembling that associated with, actual or potential tissue damage. Depending on its duration and nature, pain is divided into acute and chronic. Pain lasting for three months or less is referred to as acute, whereas pain lasting for more than three months is referred to as chronic. Acute pain is a normal protective response of the body. It occurs suddenly at the site of an injury or lesion. It generally disappears once the primary injury has healed, but it may also evolve into chronic pain under various circumstances. Chronic pain generally refers to pain that persists or recurs for more than three months. The causes and lesions are complex, and physical, blood, and imaging examinations often produce inconsistent results. The current classification system is relatively complex, with seven types identified, and it is often accompanied by emotional and psychological disorders.

Pain is currently the third major health problem after cardiovascular and cerebrovascular diseases and tumours. Not only does pain cause physical suffering to patients, it also often causes psychological stress and emotional disorders, and increases the occurrence of complications. Statistically, there are currently over 300 million people suffering from chronic pain in China, with this figure increasing by 10 to 20 million each year. There is a trend of rapid increase and younger age of onset, imposing heavy economic, physical and psychological burdens on patients and their families. Given the different pathologies and mechanisms of action of acute and chronic pain, the treatment methods and medications are also different. According to the WHO Three-Step Analgesic Ladder principle, Class I medications are the first choice, such as non-steroidal anti-inflammatory drugs (NSAIDs) or paracetamol, the latter of which has fewer adverse reactions than NSAIDs; Class II medications may be selected from weak opioids, such as tramadol; as Class III medications, strong opioids may be used, but the dosage and adverse reactions should be controlled, and addiction should be prevented. Dosage forms that have been developed for the treatment of pain include oral tablets, intravenous injections, and nasal sprays for administration via the nasal cavity. These provide multiple treatment regimens for pain patients and improve efficacy and patient compliance.

Compound A has polypeptide-like properties, is unstable at high temperatures, and is prone to producing impurities. Polypeptide compounds have disadvantages, such as poor stability when administered orally. Therefore, the stability of the preparation needs to be given particular consideration during the dosage form development process. Meanwhile, attention should be paid to controlling process parameters and the production environment during the preparation process to ensure the stable quality of raw materials and the safety and efficacy of the final product.

CN 115025205 A discloses an injection of compound A. Although intravenous injections offer rapid onset of action, chronic pain persists and recurs repeatedly, requiring long-term administration. Compared with frequent injections, oral administration is more convenient and improves compliance. Given the potent analgesic and antipruritic effects of compound A, along with the refractory nature of chronic pain which requires a prolonged cycle of treatment, developing an oral medicament of Compound A that is easy to administer, rapid in action, well absorbed, minimally irritating, stable in quality, and safe can alleviate patients' pain and pruritus while offering diversified therapeutic options for the patients.

### Summary of the Invention

An objective of the present invention is to provide an oral pharmaceutical composition of compound A, which is prepared by combining compound A with a filler or other inactive ingredients. The composition of the present invention is stable in quality, rapid in action, well absorbed and minimally irritating, and has good safety.

The present invention provides an oral pharmaceutical composition of compound A for treating pain and pruritus, which is prepared with a reasonable combination of compound A as an active ingredient and a pharmaceutically acceptable auxiliary material.

One object of the present invention is to provide an oral pharmaceutical composition of compound A.

Another object of the present invention is to provide a method for preparing the oral pharmaceutical composition.

Another object of the present invention is to provide a packaged combination of the oral pharmaceutical composition.

In one aspect, the present invention provides an oral pharmaceutical composition, comprising:
a) compound A as represented below: 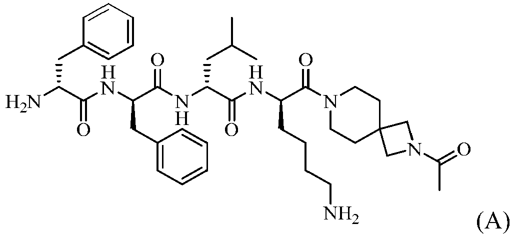 and
b) a filler selected from one or more of a microcrystalline cellulose colloidal silica co-treatment substance and pregelatinized starch.

In some embodiments, the compound A is present in the pharmaceutical composition in an amount of 0.1% to 25% w/w.

In some embodiments, the compound A is present in the pharmaceutical composition in an amount of 0.1% to 12% w/w.

In some embodiments, the compound A is present in the pharmaceutical composition in an amount of 0.1% to 9% w/w.

In some embodiments, the composition also comprises an additional filler selected from one or more of mannitol, microcrystalline cellulose, lactose, calcium hydrogen phosphate and maltodextrin.

In some embodiment, the additional filler is selected from one or more of microcrystalline cellulose and mannitol.

In some embodiments, the pharmaceutical composition further comprises one or more of a disintegrant and a lubricant.

In some embodiments, the disintegrant is selected from one or more of crospovidone, low-substituted hydroxypropyl cellulose, croscarmellose sodium, and carboxymethyl starch sodium.

In some embodiments, the disintegrant is crospovidone.

In some embodiments, the lubricant is selected from one or more of magnesium stearate and sodium stearyl fumarate.

In some embodiments, the lubricant is magnesium stearate.

In some embodiments, the filler is present in the pharmaceutical composition in an amount of 75% to 98% w/w.

In some embodiments, the filler is present in the pharmaceutical composition in an amount of 87% to 95% w/w.

In some embodiments, the disintegrant is present in the pharmaceutical composition in an amount of 0% to 5% w/w.

In some embodiments, the disintegrant is present in the pharmaceutical composition in an amount of 2% to 5% w/w.

In some embodiments, the disintegrant is present in the pharmaceutical composition in an amount of 2% to 3% w/w.

In some embodiments, the lubricant is present in the pharmaceutical composition in an amount of 0.1% to 5% w/w.

In some embodiments, the lubricant is present in the pharmaceutical composition in an amount of 0.1% to 2% w/w.

In some embodiments, the lubricant is present in the pharmaceutical composition in an amount of 0.25% to 2% w/w.

In some embodiments, the lubricant is present in the pharmaceutical composition in an amount of 0.25% to 1% w/w.

In a particular embodiment, the pharmaceutical composition comprises compound A and a microcrystalline cellulose colloidal silica co-treatment substance.

In a particular embodiment, the pharmaceutical composition comprises compound A, a microcrystalline cellulose colloidal silica co-treatment substance and pregelatinized starch.

In a particular embodiment, the pharmaceutical composition comprises compound A, a microcrystalline cellulose colloidal silica co-treatment substance and pregelatinized starch, wherein the weight ratio of the microcrystalline cellulose colloidal silica co-treatment substance to pregelatinized starch is 1 : 0.7-1.2, preferably 1 : 0.8-1.1, more preferably 1 : 0.8-1.0.

In a particular embodiment, the pharmaceutical composition comprises compound A, a microcrystalline cellulose colloidal silica co-treatment substance, a disintegrant and a lubricant.

In a particular embodiment, the compound A is present in the pharmaceutical composition in an amount of 0.1% to 25% w/w.

In a particular embodiment, the compound A is present in the pharmaceutical composition in an amount of 0.1% to 12% w/w.

In a particular embodiment, the compound A is present in the pharmaceutical composition in an amount of 0.1% to 9% w/w.

In a particular embodiment, the compound A is present in the pharmaceutical composition in an amount of 0.1% to 8% w/w.

In a particular embodiment, the compound A is present in the pharmaceutical composition in an amount of 0.1% to 7% w/w.

In a particular embodiment, the compound A is present in the pharmaceutical composition in an amount of 0.1% to 6% w/w.

In a particular embodiment, the compound A is present in the pharmaceutical composition in an amount of 0.1% to 5% w/w.

In a particular embodiment, the compound A is present in the pharmaceutical composition in an amount of 0.1% to 4% w/w.

In a particular embodiment, the compound A is present in the pharmaceutical composition in an amount of 0.1% to 3% w/w.

In a particular embodiment, the compound A is present in the pharmaceutical composition in an amount of 0.1% to 2% w/w.

In a particular embodiment, the compound A is present in the pharmaceutical composition in an amount of 0.55% w/w.

In a particular embodiment, the compound A is present in the pharmaceutical composition in an amount of 2.22% w/w.

In a particular embodiment, the compound A is present in the pharmaceutical composition in an amount of 8.8% w/w.

In a particular embodiment, the compound A is present in the pharmaceutical composition in an amount selected from: 0.1%-1.5% w/w, 2%-3% w/w, 8%-9% w/w, 8%-10% w/w and 0.1%-10% w/w.

In a particular embodiment, the pharmaceutical composition comprises compound A, a microcrystalline cellulose colloidal silica co-treatment substance, pregelatinized starch, crospovidone, and magnesium stearate.

In a particular embodiment, the pharmaceutical composition comprises compound A, a microcrystalline cellulose colloidal silica co-treatment substance, pregelatinized starch, and magnesium stearate. In particular, pregelatinized starch can also be used as a disintegrant.

In a particular embodiment, the pharmaceutical composition comprises 0.1%-10% w/w of compound A, 40%-55% w/w of a microcrystalline cellulose colloidal silica co-treatment substance, 40%-50% w/w of pregelatinized starch, 2%-5% w/w of crospovidone, and 0.1%-0.5% w/w of magnesium stearate.

In a particular embodiment, the pharmaceutical composition comprises 2.2% w/w of compound A, 50% w/w of a microcrystalline cellulose colloidal silica co-treatment substance, 44.4% w/w of pregelatinized starch, 3% w/w of crospovidone, and 0.25% w/w of magnesium stearate.

In a particular embodiment, the pharmaceutical composition comprises 8.8% w/w of compound A, 43.4% w/w of a microcrystalline cellulose colloidal silica co-treatment substance, 44.4% w/w of pregelatinized starch, 3% w/w of crospovidone, and 0.25% w/w of magnesium stearate.

In a particular embodiment, the pharmaceutical composition comprises 2.3% w/w of compound A, 51.6% w/w of a microcrystalline cellulose colloidal silica co-treatment substance, 45.8% w/w of pregelatinized starch, and 0.26% w/w of magnesium stearate.

In a particular embodiment, the pharmaceutical composition comprises 2.2% w/w of compound A, 50.1% w/w of a microcrystalline cellulose colloidal silica co-treatment substance, 44.4% w/w of pregelatinized starch, 3% w/w of crospovidone, and 0.25% w/w of magnesium stearate.

In a particular embodiment, the pharmaceutical composition comprises 2.2% w/w of compound A, 49% w/w of a microcrystalline cellulose colloidal silica co-treatment substance, 43.6% w/w of pregelatinized starch, 4.9% w/w of crospovidone, and 0.25% w/w of magnesium stearate.

In a particular embodiment, the pharmaceutical composition comprises 1.1% w/w of compound A, 53.4% w/w of a microcrystalline cellulose colloidal silica co-treatment substance (dried), 44.4% w/w of pregelatinized starch (dried), and 1% w/w of magnesium stearate.

In a particular embodiment, the pharmaceutical composition comprises 11.1% w/w of compound A, 43.4% w/w of a microcrystalline cellulose colloidal silica co-treatment substance (dried), 44.4% w/w of pregelatinized starch (dried), and 1% w/w of magnesium stearate.

In a particular embodiment, the pharmaceutical composition comprises 4.4% w/w of compound A, 48% w/w of a microcrystalline cellulose colloidal silica co-treatment substance (dried), 44.4% w/w of pregelatinized starch (dried), 2-3% w/w of crospovidone, and 0.25-1% w/w of magnesium stearate.

In a particular embodiment, the pharmaceutical composition comprises 4.4% w/w of compound A, 48% w/w of a microcrystalline cellulose colloidal silica co-treatment substance (LM), 44.4% w/w of pregelatinized starch (LM), 2-3% w/w of crospovidone, and 0.25-1% w/w of magnesium stearate.

In a particular embodiment, the pharmaceutical composition comprises 4.4% w/w of compound A, 48% w/w of a microcrystalline cellulose colloidal silica co-treatment substance (LM, dried), 44.4% w/w of pregelatinized starch (LM, dried), 2-3% w/w of crospovidone, and 0.25-1% w/w of magnesium stearate.

In a particular embodiment, the pharmaceutical composition comprises 4.8% w/w of compound A and 95.2% w/w of a microcrystalline cellulose colloidal silica co-treatment substance.

In a particular embodiment, the pharmaceutical composition comprises 4.8% w/w of compound A and 95.2% w/w of pregelatinized starch.

In a particular embodiment, the pharmaceutical composition comprises 4.8% w/w of compound A and 95.2% w/w of microcrystalline cellulose.

In a particular embodiment, the pharmaceutical composition comprises 4.8% w/w of compound A and 95.2% w/w of mannitol.

The present invention relates to a pharmaceutical preparation, comprising any one of the foregoing pharmaceutical compositions.

In some embodiments, the compound A is present in a unit preparation in an amount from 0.1 mg to 10 mg.

In some embodiments, the compound A is present in a unit preparation in an amount selected from 0.5 mg, 1 mg, 2 mg, 4 mg, 8 mg or 10 mg.

In some embodiments, the unit preparation comprises the following components: 0.5 mg of compound A, 40-50 mg of a microcrystalline cellulose colloidal silica co-treatment substance, 35-45 mg of pregelatinized starch, 2.0-3.5 mg of crospovidone, and 0.15-0.3 mg of magnesium stearate.

In some embodiments, the unit preparation comprises the following components: 2 mg of compound A, 40-50 mg of a microcrystalline cellulose colloidal silica co-treatment substance, 35-45 mg of pregelatinized starch, 2.0-3.5 mg of crospovidone, and 0.15-0.3 mg of magnesium stearate.

In some embodiments, the unit preparation comprises the following components: 8 mg of compound A, 35-45 mg of a microcrystalline cellulose colloidal silica co-treatment substance, 35-45 mg of pregelatinized starch, 2.0-3.5 mg of crospovidone, and 0.15-0.3 mg of magnesium stearate.

In some embodiments, the unit preparation comprises the following components: 0.5-10 mg of compound A, 35-50 mg of a microcrystalline cellulose colloidal silica co-treatment substance, 35-45 mg of pregelatinized starch, 1-5 mg of crospovidone, and 0.1-0.5 mg of magnesium stearate.

In another aspect, the present invention provides a preparation prepared from the above-mentioned oral pharmaceutical composition and a pharmaceutically acceptable auxiliary material, wherein the preparation is in a form selected from a tablet or a capsule.

In another aspect, the present invention provides a method for preparing the above-mentioned tablet, the method comprising the steps of: mixing compound A with an auxiliary material uniformly, compressing the mixture into tablets, and packaging.

In another aspect, the present invention provides a packaged combination of the above-mentioned oral pharmaceutical composition, wherein the packaged combination comprises:
a) an aluminium-plastic blister package,
b) a desiccant,
c) a composite bag; and
d) the oral pharmaceutical composition or pharmaceutical preparation of the present invention,

wherein the blister plate of the aluminium-plastic blister package includes a hard sheet and a pharmaceutically acceptable aluminium foil, the hard sheet is selected from a hard polyvinyl chloride sheet for solid pharmaceutical packaging, a hard polyvinyl chloride/polyvinylidene chloride composite sheet for solid pharmaceutical packaging, and a hard polyvinyl chloride/polyethylene/polyvinylidene chloride composite sheet for solid pharmaceutical packaging;
the desiccant is selected from one of a molecular sieve desiccant and a silica gel desiccant; and
the composite bag is a polyester/aluminium/polyethylene composite bag for pharmaceutical packaging.

The oral pharmaceutical composition of the present invention is provided in a packaged combination suitable for the product that provides good moisture protection, thereby ensuring the stability of the preparation and minimizing impurity growth.

The oral pharmaceutical composition provided in the present invention can significantly improve the stability of the preparation; thus, the preparation has good stability and dissolution rate.

Unless stated to the contrary, the terms in the present application are defined as follows:
In the present invention, the term "amount" is the ratio of the component to the final pharmaceutical composition (such as tablets), wherein w/w refers to the mass-to-mass ratio, and the unit is g/g in the present application. For example, the amount of 1% for compound A means that 100 g of the pharmaceutical composition contains 1 g of compound A.

In the examples of the present invention, SMCC refers to a microcrystalline cellulose colloidal silica co-treatment substance, which is a highly functional auxiliary material based on microcrystalline cellulose, prepared by blending microcrystalline cellulose and colloidal silicon dioxide in water and then drying. Pharmaceutical-grade SMCC products are currently available on the market from multiple manufacturers.

### Detailed Description of Embodiments

The technical problems to be solved, technical solutions and beneficial effects of the present invention are described below in conjunction with examples. It should be understood that the specific examples described herein are merely used to explain the present invention, but are not intended to limit the present invention.

### Example 1: Filler screening

Preparation process: compound A was uniformly mixed with different fillers at a given mass ratio, the mixture was dispensed into penicillin vials, then the vials were placed sealed or unsealed under accelerated conditions (40°C ± 2°C/75% ± 5% RH) for 28 days, and the changes in related substances of the samples were investigated. The results are shown in Table 1.

**Table 1 Filler type screening**

| | | Formulation 1 | Formulation 2 | Formulation 3 | Formulation 4 | Formulation 5 |
|---|---|---|---|---|---|---|
| Formulation composition | | Compound A | Compound A | Compound A | Compound A | Compound A |
| | | - | SMCC | Pregelatinized starch | Microcrystalline cellulose | Mannitol |
| Formulation ratio | | - | 1:20 | 1:20 | 1:20 | 1:20 |
| Total amount of impurities (%) | 0 day | 0.34 | 0.28 | 0.21 | 0.28 | 0.28 |
| | Sealed, 28 days | 0.55 | 0.65 | 0.86 | 1.51 | 5.03 |
| | Unsealed, 28 days | 1.12 | 2.74 | 2.54 | 5.51 | 12.51 |

Results: as can be seen in Table 1, after compound A was placed under accelerated conditions (40°C ± 2°C/75% ± 5% RH) for 28 days, there was no significant growth of impurities under the sealed (water-proof and moisture-proof) condition. Under the unsealed condition, the impurities grew significantly, indicating that compound A itself is relatively sensitive to humidity.

After being placed under accelerated conditions (40°C ± 2°C/75% ± 5% RH) for 28 days, the samples of formulations 1-5 showed a similar trend in impurity growth under both unsealed and sealed conditions. The type of filler in the formulation significantly affected the stability of the samples. Growth of related substances in the sample of formulation 5 was more significant.

Under the sealed (water-proof and moisture-proof) condition, the filler pregelatinized starch and compound A achieved good stability and compatibility. Compound A was significantly more stable in the filler SMCC group than in the microcrystalline cellulose group.

### Example 2: Preparation stability investigation

Based on the results in Example 1, the impact of different fillers and auxiliary materials (with or without drying treatment) on the stability of the preparations was comparatively investigated. The formulations of the preparations are shown in Table 2 and Table 3.

Preparation process: the auxiliary materials were sieved, respectively, then compound A was mixed with the auxiliary materials uniformly, and the mixture was compressed into tablets.

For formulations 9-11, both SMCC (LM or otherwise) and pregelatinized starch (LM or otherwise) were all subjected to drying pretreatment (herein LM indicates the low-moisture type of the auxiliary material).

**Table 2 Formulations for preparation stability investigation (1)**

| | Formulation 6 | Formulation 7 | Formulation 8 |
|---|---|---|---|
| Composition | Amount (mg) | Amount (mg) | Amount (mg) |
| Compound A | 4 | 4 | 4 |
| SMCC | 43.3 | 43.075 | - |
| SMCC (LM) | - | - | 43.075 |
| Pregelatinized starch | 40 | - | - |
| Pregelatinized starch (LM) | - | 40 | 40 |
| Crospovidone | 1.8 | 2.7 | 2.7 |
| Magnesium stearate | 0.9 | 0.225 | 0.225 |
| Total | 90 | 90 | 90 |
| Initial moisture content (%) | 7.60 | - | 5.60 |

**Table 3 Formulations for preparation stability investigation (2)**

| | Formulation 9 | Formulation 10 | Formulation 11 |
|---|---|---|---|
| Composition | Amount (mg) | Amount (mg) | Amount (mg) |
| Compound A | 4 | 1 | 10 |
| Dried SMCC | - | 48.1 | 39.1 |
| Dried SMCC (LM) | 43.075 | - | - |
| Dried pregelatinized starch | - | 40 | 40 |
| Dried pregelatinized starch (LM) | 40 | - | - |
| Crospovidone | 2.7 | - | - |
| Magnesium stearate | 0.225 | 0.9 | 0.9 |
| Total | 90 | 90 | 90 |
| Initial moisture content (%) | 4.60 | 3.80 | 4.20 |

The bare tablets of the preparations of formulations 6-11 mentioned above were respectively placed under accelerated conditions (40°C ± 2°C/75% ± 5% RH, unsealed) for a 2-month stability investigation. The investigation results are shown in Tables 4 and 5 below.

**Table 4 Results of preparation (bare tablet) stability investigation (1)**

| | | Formulation 6 | Formulation 7 | Formulation 8 |
|---|---|---|---|---|
| Initial value | Total amount of impurities (%) | 0.35 | - | 0.34 |
| | Number of impurities | 4 | - | 4 |
| | Moisture content (%) | 7.60 | - | 5.60 |
| Bare tablet Accelerated condition (40°C ± 2°C/75% ± 5% RH) | Total amount of impurities (%) | 8.40 | 10.54 | 9.15 |
| | Number of impurities | 30 | 30 | 29 |
| | Moisture content (%) | 9.04 | 10.00 | 8.26 |

**Table 5 Results of preparation (bare tablet) stability investigation (2)**

| | | Formulation 9 | Formulation 10 | Formulation 11 |
|---|---|---|---|---|
| Initial value | Total amount of impurities (%) | 0.34 | 0.35 | 0.35 |
| | Number of impurities | 4 | 4 | 4 |
| | Moisture content (%) | 4.60 | 3.80 | 4.20 |
| Bare tablet Accelerated condition (40°C ± 2°C/75% ± 5% RH) | Total amount of impurities (%) | 9.09 | 15.17 | 5.05 |
| | Number of impurities | 29 | 38 | 19 |
| | Moisture content (%) | 8.22 | 8.94 | 9.08 |

Using commercially available low-moisture (LM) auxiliary materials (SMCC and pregelatinized starch) or drying the auxiliary materials prior to preparation significantly reduced the initial moisture content of the preparations. However, after two months under accelerated conditions, the moisture content and impurities increased significantly.

The above-mentioned results showed that after the reserved samples of bare tablets were placed under accelerated conditions for 2 months, both the number and total amount of impurities of the samples from each formulation increased significantly. These results suggest that the stability of the preparation can be improved by strengthening the product's packaging to protect it from external moisture.

The preparation samples selected based on the above-mentioned formulations were packaged in aluminium-plastic blisters and then encapsulated together with a molecular sieve desiccant in polyester/aluminium/polyethylene composite bags for pharmaceutical packaging to obtain packaged combination samples. The packaged combination samples were placed under accelerated conditions (40°C ± 2°C/75% ± 5% RH) for a 2-month stability investigation. The investigation results are shown in Tables 6 and 7 below.

**Table 6 Results of preparation (packaged combination) stability investigation (3)**

| | | Formulation 6 | Formulation 7 | Formulation 8 |
|---|---|---|---|---|
| Initial value | Total amount of impurities (%) | 0.35 | - | 0.34 |
| | Number of impurities | 4 | - | 4 |
| | Moisture content (%) | 7.60 | - | 5.60 |
| Packaged samples Accelerated condition (40°C ± 2°C/75% ± 5% RH) | Total amount of impurities (%) | 0.75 | 0.72 | 0.86 |
| | Number of impurities | 7 | 7 | 8 |
| | Moisture content (%) | 3.47 | 2.70 | 2.73 |

**Table 7 Results of preparation (packaged combination) stability investigation (4)**

| | | Formulation 9 | Formulation 10 | Formulation 11 |
|---|---|---|---|---|
| Initial value | Total amount of impurities (%) | 0.34 | 0.35 | 0.35 |
| | Number of impurities | 4 | 4 | 4 |
| | Moisture content (%) | 4.60 | 3.80 | 4.20 |
| Packaged samples Accelerated condition (40°C ± 2°C/75% ± 5% RH) | Total amount of impurities (%) | 0.94 | 0.76 | 0.71 |
| | Number of impurities | 7 | 6 | 6 |
| | Moisture content (%) | 2.25 | 1.55 | 1.83 |

As can be seen from the results in Tables 6 and 7, the desiccant can effectively control the moisture content of the tablets of the formulations in packaged combinations to a lower level, regardless of the initial moisture levels of the preparations. The impurity growth trend of all samples was also significantly slowed down. The stability of the preparations was also significantly improved.

### Example 3: Sample quality study

The manufacturing process and the uniformity of the finished preparation product were investigated for the preparations of compound A with a low preparation strength and a compound A content of less than 25% of the unit weight.

With reference to the technical guidelines, such as the Technical Guidelines for the Study of Powder Mix Uniformity and In-Process Dosage Unit Uniformity for Chemical Oral Solid Preparations (Trial Implementation) issued by the Centre for Drug Evaluation of the National Medical Products Administration of the People's Republic of China, the powder mix uniformity of the total mixed drug powder was investigated. The content uniformity of the preparations was tested with reference to the General Chapter 0941 Content Uniformity Test in Volume IV of the Chinese Pharmacopoeia (2020 Edition).

Preparation process: the auxiliary materials were sieved, respectively, then compound A was mixed with the auxiliary materials uniformly, and the mixture was compressed into tablets.

The specific formulations and the test results are shown in Table 8 below.

**Table 8 Sample quality study**

| | | Formulation 17 | Formulation 12 | Formulation 13 |
|---|---|---|---|---|
| Composition | | Amount (mg) | Amount (mg) | Amount (mg) |
| Compound A | | 0.5 | 2 | 8 |
| SMCC | | 46.575 | 45.075 | 39.075 |
| Pregelatinized starch | | 40 | 40 | 40 |
| Crospovidone | | 2.7 | 2.7 | 2.7 |
| Magnesium stearate | | 0.225 | 0.225 | 0.225 |
| Total | | 90 | 90 | 90 |
| Powder mix uniformity | Mean (%) | 0.54 | 2.2 | 8.8 |
| | RSD (%) | 0.8 | 1.2 | 0.7 |
| Content uniformity | A+2.2S | 3.9 | 3.8 | 4.9 |

Results: As can be seen in Table 8, the RSD for the powder mix uniformity of the total mixed drug powder was less than 5%; in terms of the content uniformity of the finished product, A+2.2S ≤ 15.0. Both the powder mix uniformity and content uniformity met the relevant requirements. The manufacturing process and the sample quality were stable.

### Example 4: Dissolution curve

According to the Dissolution and Drug Release Test (General Chapter 0931 Method 2 in Volume IV of the Chinese Pharmacopoeia (2020 Edition)), the dissolution of the samples in pH 1.0 buffer solutions (at a rotation speed of 50 rpm) was investigated. Specific results are shown in Table 9 below.

**Table 9 Dissolution curve**

| | | Formulation 14 | Formulation 15 | Formulation 16 |
|---|---|---|---|---|
| Composition | | Amount (mg) | Amount (mg) | Amount (mg) |
| Compound A | | 2 | 2 | 2 |
| SMCC | | 45.075 | 45.075 | 45.075 |
| Pregelatinized starch | | 40 | 40 | 40 |
| Crospovidone | | 0 | 2.7 | 4.5 |
| Magnesium stearate | | 0.225 | 0.225 | 0.225 |
| Total | | 87.3 | 90 | 91.8 |
| Dissolution rate (%) | 5 min | 76 | 98 | 94 |
| | 10 min | 93 | 99 | 95 |
| | 15 min | 96 | 99 | 95 |
| | 30 min | 97 | 100 | 95 |

Preparation process: the auxiliary materials were sieved, respectively, then compound A was mixed with the auxiliary materials uniformly, and the mixture was compressed into tablets.

Results: it can be seen in Table 9 that the samples dissolved rapidly, achieving a dissolution rate of greater than 85% at 15 min.

In summary, the samples of the pharmaceutical composition of the present invention were prepared by mixing compound A with specific fillers, and the samples were then packaged in specific packaged combinations. The preparation process of the composition is simple and stable, and the product obtained has uniform content, consistent quality, and good stability.

### Example 5: Pharmacokinetic investigation

After a single oral administration of the preparation samples obtained in Example 3 to male Beagle dogs (3 for each preparation), venous blood was taken at 0, 0.0833, 0.25, 0.5, 1, 2, 4, 6, 8, 10, 12 and 24 h, and the pharmacokinetic parameters were calculated from the area under the curve obtained from the points where the plasma concentration of compound A was taken as a function of time; tests for an intravenous injection group were conducted in parallel. The pharmacokinetic results of the two groups are shown in Table 8:

**Table 10 Results of pharmacokinetic investigation in dogs**

| Test medicaments | Administration mode | Dose | Tₘₐₓ (h) | Cmax (ng/ ml) | AUC (h·ng·mL⁻¹) |
|---|---|---|---|---|---|
| Compound A injection | Intravenous injection | 3 mg | - | 1807 ± 281 | 2106 ± 325 |
| Formulation 12 | Oral administration | 2 mg | 1.33 ± 0.58 | 3.57 ± 2.14 | 27.4 ± 24.1 |
| Formulation 13 | Oral administration | 8 mg | 1.33 ± 0.58 | 18.9 ± 21 | 83.1 ± 45 |

As calculated based on the results in Table 10, comparable *in vivo* exposure was achieved in Beagle dogs following a single oral administration of 2 mg to 8 mg or a single intravenous administration of 0.1 mg of compound A. These results demonstrate that the therapeutic effect of oral administration of compound A is equivalent to that of intravenous administration.

## Claims

1. An oral pharmaceutical composition, **characterised in that** the oral pharmaceutical composition comprises:
a) compound A, 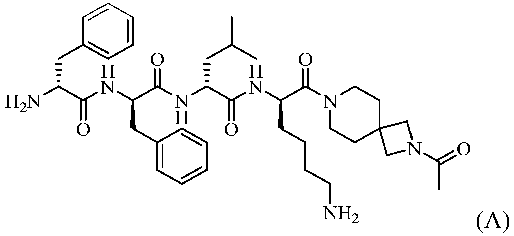 and
b) a filler selected from one or more of a microcrystalline cellulose colloidal silica co-treatment substance and pregelatinized starch, preferably a microcrystalline cellulose colloidal silica co-treatment substance.

2. The composition according to claim 1, **characterised in that** the composition comprises the compound A, a microcrystalline cellulose colloidal silica co-treatment substance and pregelatinized starch.

3. The composition according to claim 1 or 2, **characterised in that** the composition further comprises an additional filler selected from one or more of mannitol, microcrystalline cellulose, lactose, calcium hydrogen phosphate and maltodextrin, preferably one or more of microcrystalline cellulose and mannitol.

4. The composition according to any one of claims 1-3, **characterised in that** the composition further comprises one or more of a disintegrant and a lubricant.

5. The composition according to claim 4, **characterised in that**
the disintegrant is selected from one or more of crospovidone, low-substituted hydroxypropyl cellulose, croscarmellose sodium and carboxymethyl starch sodium, preferably crospovidone;
and the lubricant is selected from one or more of magnesium stearate and sodium stearyl fumarate, preferably magnesium stearate.

6. The composition according to claim 4, **characterised in that** the composition comprises the compound A, a microcrystalline cellulose colloidal silica co-treatment substance, a filler, a disintegrant, and a lubricant.

7. The composition according to claim 4, **characterised in that** the composition comprises the compound A, a microcrystalline cellulose colloidal silica co-treatment substance, pregelatinized starch, a disintegrant, and magnesium stearate.

8. The composition according to claim 4, **characterised in that** the composition comprises the compound A, a microcrystalline cellulose colloidal silica co-treatment substance, pregelatinized starch, crospovidone, and magnesium stearate.

9. The composition according to any one of claims 4-8, **characterised in that** the disintegrant is present in the pharmaceutical composition in an amount of 0% to 5% w/w.

10. The composition according to claim 9, **characterised in that** the lubricant is present in the pharmaceutical composition in an amount of 0.25% to 2% w/w.

11. The composition according to any one of claims 1-10, **characterised in that** the filler is present in the pharmaceutical composition in an amount of 75% to 98% w/w, preferably 87% to 95% w/w.

12. The composition according to any one of claim 11, **characterised in that** the compound A is present in the pharmaceutical composition in an amount of 0.1% to 25% w/w, preferably 0.1% to 12% w/w, more preferably 0.1% to 9% w/w.

13. A pharmaceutical preparation made from the oral pharmaceutical composition according to any one of claims 1-12.

14. The pharmaceutical preparation according to claim 13, **characterised in that** the compound A is present in a unit preparation thereof in an amount from 0.1 mg to 10 mg.

15. The pharmaceutical preparation according to claim 14, **characterised in that** the compound A is present in the unit preparation thereof in an amount selected from 0.5 mg, 1 mg, 2 mg, 4 mg, 8 mg or 10 mg.

16. The pharmaceutical preparation according to claim 14 or 15, **characterised in that** the unit preparation thereof comprises the following components: 2 mg of the compound A, 40-50 mg of a microcrystalline cellulose colloidal silica co-treatment substance, 35-45 mg of pregelatinized starch, 2.0-3.5 mg of crospovidone, and 0.15-0.3 mg of magnesium stearate.

17. The pharmaceutical preparation according to claim 14 or 15, **characterised in that** the unit preparation thereof comprises the following components: 8 mg of the compound A, 35-45 mg of a microcrystalline cellulose colloidal silica co-treatment substance, 35-45 mg of pregelatinized starch, 2.0-3.5 mg of crospovidone, and 0.15-0.3 mg of magnesium stearate.

18. The pharmaceutical preparation according to any one of claims 13-17, **characterised in that** the preparation is in a form selected from a tablet or a capsule.

19. A method for preparing the pharmaceutical preparation according to any one of claims 13-18, **characterised in that** the preparation is in form of a tablet, prepared by a method comprising the steps of:
(1) mixing the compound A with an auxiliary material uniformly; and
(2) compressing the mixture into tablets.

20. A packaged combination comprising the pharmaceutical composition according to any one of claims 1-12 or the pharmaceutical preparation according to any one of claims 13-18, **characterised in that** the packaged combination comprises:
a) an aluminium-plastic blister package;
b) a desiccant;
c) a composite bag; and
d) the pharmaceutical composition according to any one of claims 1-12 or the pharmaceutical preparation according to any one of claims 13-18.

21. The packaged combination according to claim 20, **characterised in that**
the blister plate of the aluminium-plastic blister package includes a hard sheet and a pharmaceutically acceptable aluminium foil, the hard sheet is selected from a hard polyvinyl chloride sheet for solid pharmaceutical packaging, a hard polyvinyl chloride/polyvinylidene chloride composite sheet for solid pharmaceutical packaging, and a hard polyvinyl chloride/polyethylene/polyvinylidene chloride composite sheet for solid pharmaceutical packaging; the desiccant is selected from a molecular sieve desiccant and a silica gel desiccant; and the composite bag is a polyester/aluminium/polyethylene composite bag for pharmaceutical packaging.
